# EUROPÄISCHE PATENTSCHRIFT

(11) **EP 1 783 508 B1**
(45) Veröffentlichungstag und Bekanntmachung des Hinweises auf die Patenterteilung: **08.10.2008**
(21) Anmeldenummer: 05024069.6
(22) Anmeldetag: 04.11.2005
(51) Int. Cl.: G01R 33/58

(54) **Testobjekt für Kernspintomographen**
Test object for nuclear magnetic resonance device
Object à tester pour un appareil à résonance magnétique nucléaire

(43) Veröffentlichungstag der Anmeldung: 09.05.2007
(73) Patentinhaber: Schleifring und Apparatebau GmbH, 82256 Fürstenfeldbruck (DE)
(72) Erfinder: Schilling, Harry, 85072 Eichstätt (DE)
(74) Vertreter: Lohr, Georg

(56) Entgegenhaltungen:
- DE-A1- 19 904 635
- FR-A- 2 708 775
- PATENT ABSTRACTS OF JAPAN Bd. 2003, Nr. 12, 5. Dezember 2003 (2003-12-05) -& JP 2004 275682 A (UEDA SENI KAGAKU SHINKOKAI; KOBAYASHI SHUNICHI; DAVID N KUU), 7. Oktober 2004 (2004-10-07)
- WEI SHAO ET AL: "Deformable registration for integration of MRI/MRSI information in TRUS-guided prostate biopsy" PROCEEDINGS OF THE SPIE - THE INTERNATIONAL SOCIETY FOR OPTICAL ENGINEERING SPIE-INT. SOC. OPT. ENG USA, Bd. 5747, Nr. 1, April 2005 (2005-04), Seiten 1263-1273, XP002370563 ISSN: 0277-786X
- MIZOWAKI T ET AL: "Towards integrating functional imaging in the treatment of prostate cancer with radiation: the registration of the MR spectroscopy imaging to ultrasound/CT images and its implementation in treatment planning" INTERNATIONAL JOURNAL OF RADIATION ONCOLOGY BIOLOGY PHYSICS ELSEVIER USA, Bd. 54, Nr. 5, 1. Dezember 2002 (2002-12-01), Seiten 1558-1564, XP002370564 ISSN: 0360-3016
- PATENT ABSTRACTS OF JAPAN Bd. 013, Nr. 293 (C-615), 6. Juli 1989 (1989-07-06) -& JP 01 086952 A (TOSHIBA CORP), 31. März 1989 (1989-03-31)
- REYNIER CHRISTOPHE ET AL: "MRI/TRUS data fusion for prostate brachytherapy. Preliminary results" MEDICAL PHYSICS, AIP, MELVILLE, NY, US, Bd. 31, Nr. 6, Juni 2004 (2004-06), Seiten 1568-1575, XP012074930 ISSN: 0094-2405
- FIRLE E A ET AL: "Mutual-information-based registration for ultrasound and CT datasets" PROCEEDINGS OF THE SPIE - THE INTERNATIONAL SOCIETY FOR OPTICAL ENGINEERING SPIE-INT. SOC. OPT. ENG USA, Bd. 5370, Nr. 1, Mai 2004 (2004-05), Seiten 1130-1138, XP002370573 ISSN: 0277-786X

## Beschreibung

### Technisches Gebiet

Die Erfindung bezieht sich auf Testobjekte für die Kernspintomographie und insbesondere auf ein Phantom zur untersuchung in keinspintomographen, umfassend einen flexiblen Probekörper in Form einer menschlichen Prostata, der sich in einem Behälter befindet. Die Kernspintomographie wird auch als Magnetresonanztomographie, abgekürzt MRT, bezeichnet.

### Stand der Technik

Zum Einsatz in der Kernspintomographie sind verschiedene Testobjekte bekannt. Diese Testobjekte werden häufig auch als Phantom bezeichnet.

So offenbart die US 6,720,766 B2 ein Testobjekt, welches unterschiedliche Strukturen im verschiedenen Raumachsen aufweist. Mit einem derartigen Testobjekt können Auflösung, Kontrast und Genauigkeit kontrolliert werden.

In der US 2005/0110490 A1 ist ein aktives Testobjekt offenbart, welches ein resonanzfähiges Spulensystem aufweist, das gesteuert in das Feld des Kernspintomographen eingreifen kann.

Die US 2005/0139758 A1 offenbart eine Nachbildung von Gelenken des menschlichen Körpers.

In PATENT ABSTRACTS OF JAPAN Bd. 2003, Nr. 12, 5. Dezember 2003 (2003-12-05) -& JP 2004 275682 A (UEDA SENI KAGAKU SHINKOKAI; KOBAYASHI SHUNICHI; DAVID N KUU), 7. Oktober 2004 (2004-10-07) ist eine Nachbildung eines Blutgefäßes für Strömungsmessungen offenbart.

In WEI SHAO ET AL: "Deformable registration for integration of MRI/MRSI information in TRUS-guided prostate biopsy" PROCEEDINGS OF THE SPIE - THE INTERNATIONAL SOCIETY FOR OPTICAL ENGINEERING SPIE-INT. SOC. OPT. ENG USA, Bd. 5747, Nr. 1, April 2005 (2005-04), Seiten 1263-1273, XP002370563 ISSN: 0277-786X wird ein Phantom gemäss dem Oberbegriff von Anspruch 1 einer magnetischen Resonanzbildgebung unterzogen.

Weiterhin offenbart MIZOWAKI T ET AL: "Towards integrating functional imaging in the treatment of prostate cancer with radiation: the registration of the MR spectroscopy imaging to ultrasound/CT images and its implementation in treatment planning" INTERNATIONAL JOURNAL OF RADIATION ONCOLOGY BIOLOGY PHYSICS ELSEVIER USA, Bd. 54, Nr. 5, 1. Dezember 2002 (2002-12-01), Seiten 1558-1564, XP002370564 ISSN: 0360-3016 eine Nachbildung menschlicher Gewebe.

Die aus dem Stand der Technik bekannten Phantome sind weitgehend abstrahierte Testobjekte die überwiegend die Aufgabe der Kalibrierung von Kernspintomographen erfüllen. Untersuchungen unter den äußerst komplexen Bedingungen, wie sie im menschlichen Körper bestehen sind mit derartigen Testobjekten nicht möglich. Diese müssen entsprechend dem Stand der Technik nach wie vor an Testpersonen durchgeführt werden.

### Darstellung der Erfindung

Aufgabe der Erfindung ist es, ein Phantom vorzustellen, welches die tatsächlichen Gegebenheiten im menschlichen Körper mit hinreichender Genauigkeit simuliert, so dass realistische Messungen durchgeführt und Operationstechniken und Untersuchungstechniken ausprobiert werden können. Insbesondere soll mit einer erfindungsgemäßen Vorrichtung eine möglichst realistische Nachbildung der Prostata sowie deren Umgebung möglich sein, welche reproduzierbare Ergebnisse liefert und gleichzeitig kostengünstig herstellbar ist.

Eine erfindungsgemäße Lösung dieser Aufgabe ist in Anspruch 1 gegeben. Weiterbildungen der Erfindung sind Gegenstand der abhängigen Ansprüche. Anspruch 9 definiert ein Verfahren zur Verwendung eines erfindungsgemässen Phantoms.

Ein erfindungsgemäßes Phantom 1 zur Untersuchung in Kernspintomographen umfasst einen Probekörper 3 zur abstrahierten Nachbildung einer menschlichen Prostata.

Die Position des Probekörpers 3 innerhalb des Phantoms ist veränderbar. Hierbei wird in vorteilhafter Weise die Position von außen eingestellt. So kann der Probekörper 3 in Form einer menschlichen Prostata an einem Schlauch 7 befestigt sein, der durch ein Außengewinde in seiner Position relativ zum Behälter 6 verstellbar ist. Dadurch kann auch mittelbar die Dicke der Lipidschicht eingestellt werden.

Wenn in einem Phantom gemäss Anspruch 2 die leitungen zur Nachbildung von blutgefässen von einer Flüssigkeit durchströmt werden, können die Effekte (Strömungsartefakte) einer sich bewegenden Flüssigkeit auf eine kernspintomographische Untersuchung reproduzierbar untersucht werden.

Bevorzugt ist der Probekörper wenigstens teilweise elastisch verformbar ausgebildet von einer weiteren flexiblen Schicht umgeben. Mit dieser Schicht soll eine typische Lipidschicht nachgebildet werden.

Mit dem erfindungsgemäßen Phantom sind Feinabstimmungen der Resonanzen sowie quantitative Begutachtungen neuer Spulen und Systeme möglich. Es kann die Erforschung und Optimierung neuer Sequenzen sowie die klinische Qualitätssicherung verbessert und kostengünstiger gestaltet werden.

Das Resonanzverhalten der in Kernspintomographen eingesetzten Spulen ist sehr stark von der Geometrie der zu untersuchenden Körper sowie deren elektrischen Eigenschaften abhängig. Für die Konstruktion und Feinabstimmung der Spulen ist es daher erforderlich, die auftretenden elektrischen Gegebenheiten (resistiv, kapazitiv und induktiv) möglichst naturgetreu und gleichzeitig reproduzierbar zu simulieren.

Grundsätzlich ermöglicht ein Phantom reproduzierbare Messergebnisse. Es stellt einen objektiven Standard für die Referenzierung neuer Methoden und Verfahren sowie für die Implementierung neuer Hardware dar. Mit Hilfe eines Phantoms ist ein direkter Vergleich veränderter Parameter und Sequenzen, verschiedener Systeme, Spulen und Feldstärken möglich.
Ein Phantom mit einer definierten Zusammensetzung und bekannten geometrischen Formen.ist auch für die Qualitätssicherung unabdingbar. Bei klinischen Studien sollten die technischen Systeme regelmäßig kalibriert werden, um vergleichbare Messergebnisse absichern zu können. Dies ist nur mit standardisierten Modellen möglich.

Die Außenmaße des erfindungsgemäßen Phantoms sowie die Abmessungen der Probekörper 2, 3 entsprechen vorzugsweise den tatsächlichen Abmessungen durchschnittlicher Menschen. Die gesamten Körpermasse basieren vorteilhafterweise auf den entsprechenden Normen.

Weiterhin ist der Probekörper vorteilhafterweise in einer realistischen Relation zu anderen Körperteilen angebracht. So sollte der Probekörper in Form einer Prostata bis zu 30% der Nachbildung des Rektums überdecken und dabei einen Abstand von 1 mm bis 2 mm haben. Der sich ergebende Spalt zwischen der Nachbildung der der Prostata und der Nachbildung des Rektums ist mit einer Lipidschicht 4 gefüllt. Bei der Nachbildung ist es wichtig, dass sowohl die Nachbildung 2 des Rektums als auch die Nachbildung 3 der Prostata flexibel aber formstabil sind. Durch diese Anordnung ergibt sich entsprechend dem tatsächlichen menschlichen Körper die flexible Verbindung der beiden Teile ohne eine starre Kopplung. Hierzu können beispielsweise flexible Kunststoffe, wie dünnes PVC eingesetzt werden.

Eine weitere vorteilhafte Ausgestaltung der Erfindung sieht wenigstens eine zusätzliche Nachbildung 2 wenigstens eines weiteren Körperteils wie Rektum oder Beckenknochen vor. Durch die Integration zusätzlicher Körperteile kann eine noch realistischere Nachbildung erreicht werden.

In einer anderen vorteilhaften Ausgestaltung der Erfindung ist wenigstens eine Nachbildung eines weiteren Körperteils oder ein Probekörper durch Gewebenachbildungen mechanisch mit anderen Teilen oder auch Probekörpern verbunden, so dass die mechanische Kopplung einem natürlichen Körper entspricht. Dadurch können auch bestimmte Bewegungsvorgänge des Patienten sowie beim einführen von Instrumenten, beispielsweise von Rektalsonden nachgebildet werden.

In einer weiteren vorteilhaften Ausgestaltung ist die Nachbildung 3 einer Prostata innerhalb eines komplexen Phantoms 1, welches beispielsweise eine vollständige Beckenregion nachbildet, austauschbar. Damit kann die Variation einzelner Teile in einem ansonsten reproduzierbar konstanten Umfeld untersucht werden. Beispielsweise können die unterschiedlichen Eigenschaften einer vergrößerten gegenüber einer kleinen, einer tumorösen gegenüber einer gesunden, einer fettreichen gegenüber einer fettarmen Prostata studiert und erprobt werden.

Alternative Probekörper könnten das entsprechende Organ beispielsweise durch einen Quader, einen Zylinder oder andere geometrische Körper ersetzen. Dies ist keine sehr realistische Nachbildung, mit der keine realen Anwendungen untersucht werden können. Allerdings können mit einfachen geometrischen Formen Kalibrierungen einfacher durchgeführt werden, da die Lage von Ecken und kanten sowie anderen geometrischen Merkmalen genau bekannt ist. Weiterhin sind geometrische Körper meist einfacher zu fertigen.

Ein Verfahren zur Herstellung von Probekörpern, vorzugsweise in Form einer Prostata umfasst die Schritte Eintauchen einer Form in flüssigen Kunststoff und anschließendes Aushärten. Die Form zur Herstellung eines Probekörpers kann beispielsweise durch Stereolithographie (Rapid-Prototyping) oder auch durch ein LOM-Verfahren oder CNC-Fräsen hergestellt sein. Vorzugsweise kann der Probekörper danach mit einem Füllmaterial zur Simulierung des Gewebes, beispielsweise Gelatine mit Zusätzen, gefüllt werden. Hierzu wird der Probekörper vorzugsweise in einem Behälter mit einem Material wie Wasser oder Glaskügelchen zur Erzeugung eines Gegendrucks gelagert. Dadurch können Verformungen während der Befüllung vermieden werden.

Entsprechend kann auch der Fettmantel (Lipidschicht) eines Organs oder eines Körperteiles, insbesondere der Prostata nachgebildet werden. Hierzu wird der Probekörper des Organs beziehungsweise der Prostata in eine entsprechende Flüssigkeit eingetaucht, welche sich anschließend verfestigt beziehungsweise aushärtet.

Eine erfindungsgemäßes Phantom 1 ist vorzugsweise in wenigstens zwei Teilen gefertigt. Hierbei ergibt der obere Teil des Phantoms hauptsächlich der elektromagnetischen Belastung der Spulen. Der untere Teil des Phantoms umfassend Rektum und Prostata enthält alle wesentlichen Komponenten zur spektroskopischen und bildgebenden Qualitätsmessung. Vorteilhafterweise sind alle Komponenten so ausgelegt, dass sie vakuumstabil sind und leicht entgast werden können. Zusätzlich werden die Komponenten so gestaltet, dass sie leicht füllbar und innerhalb des Phantoms ebenso leicht austauschbar sind. Da trotz der Entgasung nicht sichergestellt werden kann, dass jegliches Gas entweicht, wird jede Komponente des Phantoms so konstruiert, dass sich eventuell verbliebenes Gas in Bereichen der Komponenten sammeln kann die für die Bildgebung nicht relevant sind. Dies sind Bereiche, welche möglichst weit von den Zentren der Probekörper entfernt sind. Die Komponenten sind vorteilhafterweise dünnwandig ausgeführt, um Suszeptibilitätssprünge zu vermeiden.

Weiterhin ist es vorteilhaft, wenn zusätzlich ein Auflösungsmuster 5a, 5b, 5c, 5d mit definierter Geometrie vorgesehen ist. Durch dieses kann dann eine exakte Kalibrierung der Ortskoordinaten und der entsprechenden Auflösung erfolgen.

Weiterhin ist es vorteilhaft, wenn an wenigstens einem Probekörper (2, 3) sichtbare Markierungen zur Positionskontrolle vorgesehen sind.

### Beschreibung der Zeichnungen

Die Erfindung wird nachstehend ohne Beschränkung des allgemeinen Erfindungsgedankens anhand von Ausführungsbeispielen unter Bezugnahme auf die Zeichnungen exemplarisch beschrieben.

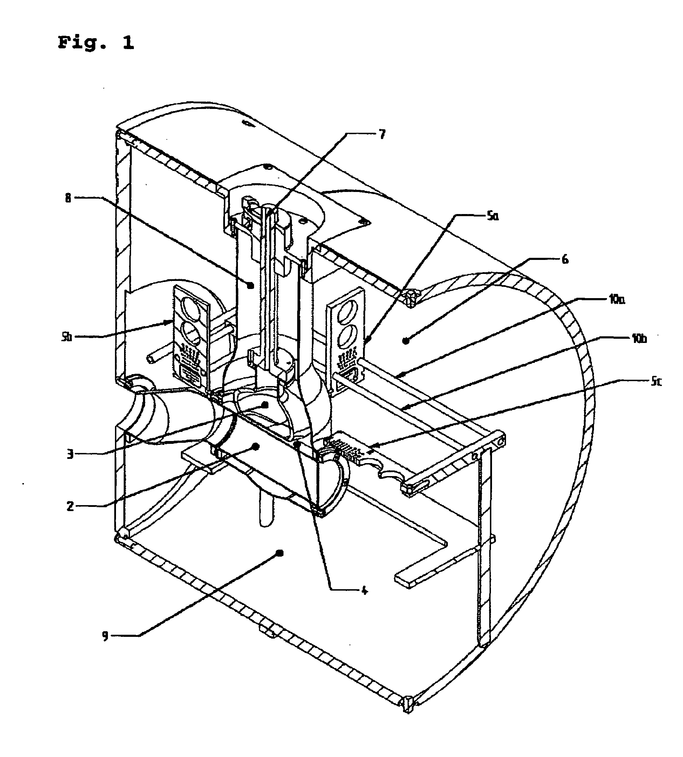

Fig. 1 zeigt beispielhaft eine erfindungsgemäße Anordnung.

Fig. 1 zeigt beispielhaft ein erfindungsgemäßes Prostataphantom 1. Innerhalb eines Behälters 6 befinden sich ein Probekörper 3 in Form einer Prostatanachbildung sowie ein weiterer Probekörper 2 in Form einer Nachbildung eines Rektums. Um die Prostatanachbildung 3 ist die Nachbildung einer Lipidschicht 4 angeordnet. Die Prostatanachbildung 3 ist mittels einer Haltevorrichtung 7, hier beispielsweise in Form eines Schlauches mit dem Behälter 6 verbunden. Diese Haltevorrichtung ist in der Lage einstellbar, so dass die Prostatanachbildung 3 in Form und Lage von außen einstellbar ist. Der Hohlraum ist in diesem Beispiel derart bemessen, dass der Prostata-Probekörper 3 durch den Hohlraum entnommen und modifiziert bzw. ausgetauscht werden kann. In den Hohlraum 8 kann eine Flüssigkeit zur Nachbildung der Lipidschicht eingebracht werden. Mittels der Auflösungsmuster 5a, 5b, 5c, 5d ist eine Kontrolle der Auflösung und eine Kalibrierung des Kernspintomographen in drei Achsen möglich. Das Innenvolumen 9 des Phantoms ist mit einer Flüssigkeit zur Belastung der Resonanzspulen des Kernspintomographen, beispielsweise eine wässrige Salzlösung gefüllt. Die Leitungen 10a, 10b dienen zur Nachbildung von Blutgefäßen und den durch die Blutströmung verursachten Strömungsartefakten. Vorzugsweise ist eine nicht darggestellte Pumpe zur Versorgung vorgesehen. Alternativ können die Leitungen auch durch Infusionsflaschen oder andere Gefäße versorgt werden. Die Leitungen sind hier als gerade Rohre und somit mit exakt definierten Positionen ausgebildet. Sie können gleichzeitig noch zu andere Funktionen, wie der Halterung von Auflösungsmustern dienen. Alternativ werden die Leitungen in der Form von im menschlichen Körper vorkommenden Blutgefäßen angeordnet.

### Bezugszeichenliste

- 1: Phantom
- 2: Probekörper Rektum
- 3: Probekörper Prostata
- 4: Nachbildung einer Lipidschicht
- 5a, 5b, 5c, 5d: Auflösungsmuster
- 6: Behälter
- 7: Aufhängung des Prostata-Probekörpers
- 8: Hohlraum
- 9: Innenvolumen
- 10: Nachbildung Blutgefäß

## Patentansprüche

1. Phantom (1) zur Untersuchung in Kernspintomographen umfassend einen flexiblen Probekörper (2, 3) zur abstrahierten Nachbildung einer menschlichen Prostata, welcher sich in einem Behälter (6) befindet,
**dadurch gekennzeichnet, dass**
der Probekörper im Innern des Behälters in einem separaten Hohlraum (8) angeordnet ist, in den eine Flüssigkeit zur Ausbildung einer Lipidschicht eingebracht werden kann, und dass der Probekörper mittels einer Haltevorrichtung (7) mit dem Behälter (6) verbunden ist, wobei die Haltevorrichtung in der Lage einstellbar ist, so dass der Probekörper in Form und Lage von außen einstellbar ist.

2. Phantom (1) nach Anspruch 1,
**dadurch gekennzeichnet, dass** Leitungen (10a, 10b) zur Nachbildung von Blutgefässen und durch die Blutströmung verursachten Strömungsartefakten vorgesehen sind.

3. Phantom (1) nach Anspruch 1,
**dadurch gekennzeichnet, dass**
eine Nachbildung eines Rektums vorgesehen ist und der Probekörper (2, 3) als Nachbildung einer Prostata von einer flexiblen Schicht (4) zur Nachbildung einer Lipidschicht umgeben ist, welche einen Spalt zwischen der Nachbildung der Prostata und der Nachbildung des Rektums füllt.

4. Phantom (1) nach einem der vorhergehenden Ansprüche,
**dadurch gekennzeichnet, dass**
wenigstens eine Nachbildung eines weiteren Körperteiles wie Rektum oder Beckenknochen vorgesehen ist.

5. Phantom (1) nach einem der vorhergehenden Ansprüche,
**dadurch gekennzeichnet, dass**
wenigstens eine Nachbildung wenigstens eines weiteren Körperteils oder des Probekörpers (2, 3) durch Gewebenachbildungen mechanisch mit anderen Teilen verbunden ist, so dass die mechanische Kopplung einem natürlichen Körper entspricht.

6. Phantom (1) nach einem der vorhergehenden Ansprüche,
**dadurch gekennzeichnet, dass**
Teile, insbesondere die Nachbildung der Prostata innerhalb des Phantoms, welches beispielsweise eine vollständige Beckenregion nachbildet, austauschbar sind.

7. Phantom (1) nach einem der vorhergehenden Ansprüche,
**dadurch gekennzeichnet, dass**
wenigstens ein Auflösungsmuster (5a, 5b, 5c, 5d) vorgesehen ist.

8. Phantom (1) nach einem der vorhergehenden Ansprüche,
**dadurch gekennzeichnet, dass**
am Probekörper (3) sichtbare Markierungen zur Positionskontrolle vorgesehen sind.

9. Verfahren zur kernspintomographischen Untersuchung an einem Phantom nach Anspruch 2, wobei die Leitungen zur Nachbildung von Blutgefässen von einer Flüssigkeit durchströmt werden.

## Claims

1. A phantom (1) for examination in magnetic resonance imagers, comprising a flexible sample body (2, 3) for abstract imitation of a human prostate gland which is located in a container (6), **characterized in that** the sample body is arranged in the interior of the container in a separate cavity (8) in which a fluid can be introduced for forming a lipid layer, and that the sample body is connected with the container (6) by means of a holding apparatus (7), with the holding apparatus being adjustable in its position so that the sample body is externally adjustable with respect to shape and position.

2. A phantom (1) according to claim 1, **characterized in that** conduits (10a, 10b) are provided for imitating blood vessels and flow artifacts caused by the blood flow.

3. A phantom (1) according to claim 1, **characterized in that** an imitation of a rectum is provided and the sample body (2, 3) as an imitation of a prostate gland is enclosed by a flexible layer (4) for recreating a lipid layer which fills a gap between the imitation of the prostate gland and the imitation of the rectum.

4. A phantom (1) according to one of the preceding claims, **characterized in that** at least one imitation of a further body part such as rectum or pelvic bone is provided.

5. A phantom (1) according to one of the preceding claims, **characterized in that** at least one imitation of at least one further body part or the sample body (2, 3) is mechanically connected by tissue imitations with other parts, so that the mechanical coupling corresponds to a natural body.

6. A phantom (1) according to one of the preceding claims, **characterized in that** parts are exchangeable, especially the imitation of the prostate gland within the phantom which imitates a complete pelvic region for example.

7. A phantom (1) according to one of the preceding claims, **characterized in that** at least one resolution pattern (5a, 5b, 5c, 5d) is provided.

8. A phantom (1) according to one of the preceding claims, **characterized in that** visible markings for position control are provided on the sample body (3).

9. A method for MRI examination on a phantom according to claim 2, with the conduits for imitating the blood vessels being flowed through by a liquid.

## Revendications

1. Fantôme (1) destiné à être examiné dans des appareils d'imagerie par résonance magnétique, comprenant un élément de test souple (2, 3) reproduisant de façon abstraite une prostate humaine, qui se trouve dans un contenant (6),
**caractérisé en ce que** l'élément de test est disposé à l'intérieur du contenant dans un compartiment (8) séparé, dans lequel un liquide peut être introduit pour représenter une couche lipidique, et **en ce que** l'élément de test est relié au contenant (6) au moyen d'un dispositif de maintien (7), lequel dispositif de maintien peut être ajusté en position de telle façon que la position et la forme de l'élément de test puissent être ajustées de l'extérieur.

2. Fantôme (1) selon la revendication 1, **caractérisé en ce que** des conduites (10a, 10b) sont prévues pour reproduire les vaisseaux sanguins et les artefacts de flux provoqués par la circulation sanguine.

3. Fantôme (1) selon la revendication 1, **caractérisé en ce qu'**une reproduction d'un rectum est prévue et l'élément de test (2, 3) reproduisant une prostate est entouré d'une couche souple (4) reproduisant une couche lipidique, qui comble un espace entre la reproduction de la prostate et la reproduction du rectum.

4. Fantôme (1) selon l'une des revendications précédentes, **caractérisé en ce qu'**au moins une reproduction d'une autre partie du corps, telle que le rectum ou les os du bassin, est prévue.

5. Fantôme (1) selon l'une des revendications précédentes, **caractérisé en ce qu'**au moins une autre reproduction d'au moins une autre partie du corps ou de l'élément de test (2, 3) est reliée mécaniquement à d'autres parties par des reproductions de tissus, de sorte que le couplage mécanique correspond à celui d'un corps naturel.

6. Fantôme (1) selon l'une des revendications précédentes, **caractérisé en ce que** des parties, en particulier la reproduction de la prostate à l'intérieur du fantôme, qui reproduit par exemple une région complète du bassin, sont interchangeables.

7. Fantôme (1) selon l'une des revendications précédentes, **caractérisé en ce qu'**au moins un modèle de résolution (5a, 5b, 5c, 5d) est prévu.

8. Fantôme (1) selon l'une des revendications précédentes, **caractérisé en ce que** des marquages visibles sont prévus sur l'élément de test (3) pour le contrôle de la position.

9. Procédé pour l'examen en imagerie par résonance magnétique d'un fantôme selon la revendication 2, dans lequel les conduites reproduisant les vaisseaux sanguins sont parcourues par un liquide.
